# EUROPEAN PATENT SPECIFICATION

(11) **EP 3 183 011 B1**
(45) Date of publication and mention of the grant of the patent: **11.12.2024**
(21) Application number: 14755057.8
(22) Date of filing: 20.08.2014
(51) Int. Cl.: A61L 2/18, A01N 25/02, A01N 59/00, C09K 5/10, A61M 1/36

(54) **HEAT TRANSFER LIQUID FOR A TEMPERATURE CONTROL DEVICE FOR EXTRACORPOREAL CIRCULATION**
WÄRMETRANSFERFLÜSSIGKEIT FÜR TEMPERATURSTEUERUNGSVORRICHTUNG FÜR EXTRAKORPORALE ZIRKULATION
LIQUIDE DE TRANSFERT DE CHALEUR POUR DISPOSITIF DE RÉGULATION DE TEMPÉRATURE POUR CIRCULATION EXTRACORPORELLE

(43) Date of publication of application: 28.06.2017
(73) Proprietor: Sorin Group Deutschland GmbH, 80939 München (DE)
(72) Inventor: KNOTT, Erwin, 85586 Poing (DE); HECKMAIER, Thomas, 80939 München (DE); DE SOMER, Filip, B-9000 Ghent (BE)
(74) Representative: Grünecker Patent- und Rechtsanwälte PartG mbB
(86) International application number: PCT/EP2014/067746
(87) International publication number: WO 2016/026525

(56) References cited:
- WO-A1-2014/026833
- WO-A2-2009/094601
- US-A- 3 064 649
- US-A- 5 871 526

## Description

### FIELD OF THE INVENTION

The present invention relates to a heat transfer liquid for use in temperature control of the human body temperature during extracorporeal circulation and a temperature control device for human bodv temperature control.

### PRIOR ART

Extracorporeal circulation of blood is used in certain surgical procedures such as during heart surgery. During the extracorporeal circulation, the body temperature of the patient can be controlled, by controlling the temperature of the blood during extracorporeal circulation. For this purpose, a patient temperature control system can be provided by means of which the temperature of the blood of the patient in the circulation can be raised or lowered. The blood thus controlled, flows through the patient and the body of the patient approaches the temperature of the blood. So as to heat or cool the blood, the temperature control system may comprise a heater and/or cooler device providing a liquid circulation to a disposable (single use) heat exchanger that transfers energy to and/or away from the patient's blood circulation. Conventionally, the liquid is water.

The heat exchanger for the blood is a strict dual circuit system, the blood side and the liquid side being separated from each other so that any mixture, such as by means of diffusion, between the blood in one of the circuits and the temperature control liquid in the other of the circuits is inhibited as much as possible. Nevertheless, care has to be taken to avoid health risks stemming from the liquid.

### RELATED ART

The applicant has designed a mobile temperature control device for human body temperature control during extracorporeal circulation. Such a mobile device can be connected to a circuit of temperature control liquid to be used in a heat exchanger. The mobile temperature control device preferably is provided with exchangeable hoses or tubes or other conduits. Further, connecting and disconnecting these conduits can more easily be achieved if the conduits are not filled with the temperature control liquid during connecting and disconnecting. Likewise, it is preferred if the circuit can be emptied of temperature control liquid for the connection and disconnection of the mobile temperature control device. The preferred mobile device is consequently provided with an open reservoir where the temperature control liquid is exposed to environmental air. The temperature control liquid can be fed into the circuit from this reservoir and can be returned to it. Any air which might be trapped in one of the conduits during connecting or disconnecting the conduit and the temperature control device or the heat exchanger can be bled to the environment via the open reservoir. This means, however, that the temperature control liquid is exposed to the air of the environment.

Substances used as temperature control liquid, in particular water, are prone to microbial contamination when exposed to environmental air. If the temperature control liquid was exposed to the environmental air, disinfecting the temperature control device and the temperature control liquid can then improve the microbial status of the liquid and render the mobile device maintenance- and service-friendly.

Usually, the microbial growth in water is rapid. The microbial population in water of drinking quality, which has a microbial population of less than 100 cfu/ml, grows within 14 days to as much as 1,000,000 cfu/ml. Therefore, it was essential for a sterile operation of extracorporeal circulation that the water used as the heat transfer liquid for human body temperature control during extracorporeal circulation was exchanged regularly.

Further, in the prior art, disinfecting the temperature control device, i.e. the heater and/or cooler, was necessary in view of the rapid growth of the microbial population in water. It was, for example, accomplished by regularly disassembling all devices and subjecting the disassembled parts to a separate disinfection procedure. This was both time consuming and expensive. Further, it was required to establish a monitoring and recordation system in order to maintain and verify the disinfection status of the heater and/or cooler system within an acceptable range.

In view of the above situation, it is presently required to spend a lot of time, effort and money on the maintenance of a temperature control device for human body temperature control during extracorporeal circulation.

Document WO 2014/026833 A1 discloses a method for controlling a disinfection status of a heater and/or cooler for human body temperature control during extracorporeal circulation. The temperature control is conducted by use of a heat exchanger and a temperature control liquid circulating through the heat exchanger and the heater and/or cooler. The method comprises using a long term disinfectant in the temperature control liquid.

Document US 3 064 649 A relates to an apparatus for controlling the temperature of blood during extracorporeal circulation.

Document US 5 871 526 A discloses a portable temperature control system which includes a thermoelectric cooler with a liquid heat exchanger and a pump for circulating the fluid through the system.

### SUMMARY OF THE INVENTION

The present invention addresses the problem of the high amount of time, effort and money to be spent in order to avoid a hygienic risk for the patient and the sterile environment in an operating room in which extracorporeal circulation is carried out.

This problem is solved by the inventive heat transfer liquid according to claim 1 and the temperature control device according to claim 4. Further advantageous features and embodiments are defined in the dependent claims.

According to the present invention, there is provided a heat transfer liquid for use in temperature control of the human body temperature during extracorporeal circulation according to claim 1.

On the basis of a heat transfer liquid consisting of ethylene glycol or propylene glycol as defined in claim 1 and water at between 65 volume-percent and 75 volume-percent, the inventors have found that a very small amount of hydrogen peroxide can be used as a long-term disinfectant which renders regular disinfecting the device or exchanging of the heat transfer liquid unnecessary. Moreover, since the heat transfer liquid for a temperature control device for human body temperature control during extracorporeal circulation may, e.g. due to a leaking device, contact or mix with the blood of the patient, it is of ultimate importance that the heat transfer liquid is non-toxic and has no detrimental effects on the health status of the patient.

Both propylene glycol and hydrogen peroxide carry the designation of the U.S. Food and Drug Administration (FDA) of "Generally Recognized As Safe" (GRAS). Both substances are also used as food additives. These substances, at least in a food grade concentration, are safe also as a disinfectant in a heat transfer liquid for extracorporeal circulation, as the inventors have found.

According to the invention, microbial contamination and, as a consequence, the need for regular disinfection and replacement of the heat transfer fluid can be avoided by using the heat transfer liquid according to the present invention. This significantly reduces the maintenance efforts for a temperature control device for human body temperature control during extracorporeal circulation and even increases the safety for the patient during extracorporeal circulation since microbial contamination is prevented at all times.

For water to be considered as sterile, filtered and demineralized water in accordance with the present invention, the initial water contamination is close to 0 cfu. This result is achieved by filtering normal tap water through a water filter having a filter mesh size of 0.2 µm. As normal bacteria and algae are sized 1µm or larger, these microorganisms are not able to pass the filter, resulting in sterile or close to sterile water after the filter process.

Preferably, the heat transfer liquid comprises ethylene glycol or propylene glycol at about 30 volume-percent. This preferred amount of ethylene glycol or propylene glycol in the heat transfer liquid allows for an optimum liquid with particular reference to its bacteriostatic properties.

It is further preferred that the hydrogen peroxide is comprised in the heat transfer liquid at about 0.02 volume-percent. This concentration of hydrogen peroxide in the heat transfer liquid ensures an effective bactericide effect while maintaining the amount of hydrogen peroxide in the heat transfer liquid at a low level.

### BRIEF DESCRIPTION OF THE DRAWINGS

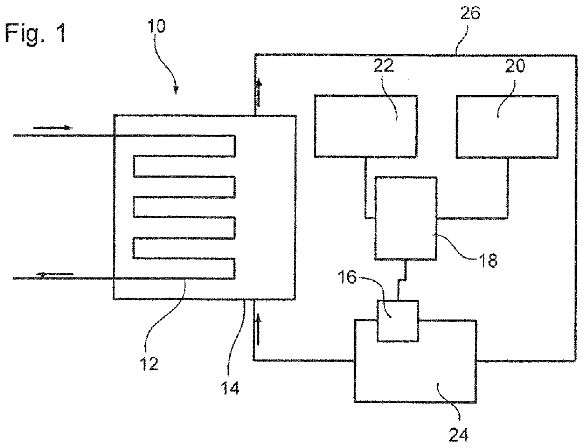

Fig. 1 shows a schematic diagram illustrating a temperature control device in a heat exchanging system.

### DETAILED DESCRIPTION OF A PREFERRED EMBODIMENT

Fig. 1 shows a schematic diagram of a heat exchanger 10 for human body temperature control during extracorporeal circulation. The heat exchanger 10 comprises a blood side 12 and a heat transfer liquid side 14 in which blood and a heat transfer liquid are respectively circulated.

The temperature of the heat transfer liquid used in the heat exchanger 10 is controlled by a heater and/or cooler 24, i.e. a temperature control device. The heater and/or cooler 24 is a device which is capable of heating or cooling or heating and cooling the heat transfer liquid. Preferably, the heater or cooler is capable of both heating and cooling the heat transfer liquid so that a defined temperature of the heat transfer liquid can be maintained. The heater and/or cooler 24 is part of a heat transfer liquid side circuit 26 in which the heat transfer liquid is circulated for operating the heat exchanger 10 in controlling the temperature of the human body of a patient.

The heater and/or cooler 24 may be provided with a disinfectant sensor 16 configured for measuring a concentration of a disinfectant in the heat transfer liquid in the heater and/or cooler 24. The result of the measurement of the sensor 16 may be transmitted to a computer 18 which may control a first display 20 which may indicate the measured concentration of disinfectant in the heat transfer liquid and a second display 22 which may indicate the consequence of the measured concentration. In particular, the second display 22 may indicate whether the disinfection status of the heat transfer liquid is ok, whether disinfectant is to be added or whether the heat transfer liquid is to be exchanged.

The heat transfer liquid circulated in the heat transfer liquid side circuit 26 of the present embodiment consists of ethylene glycol or propylene glycol at 30 volume-percent, hydrogen peroxide at 0.02 volume-percent and sterile, filtered and de-mineralized water as rest. In particular the hydrogen peroxide may be considered by the sensor 16 as disinfectant and a concentration of hydrogen peroxide may be measured by the sensor 16, But also a concentration of ethylene glycol or propylene glycol can be determined by the sensor 16 or a different sensor.

Measuring the concentration of disinfectant, be it hydrogen peroxide, ethylene glycol or propylene glycol is, however, optional and not essential for the present invention.

## Claims

1. Heat transfer liquid for use in temperature control of the human body temperature during extracorporeal circulation, the heat transfer liquid consisting of ethylene glycol at 25 volume-percent to 35 volume-percent, or propylene glycol at 30 volume-percent to 35 volume-percent,
hydrogen peroxide at 0.01 volume-percent to 0.05 volume-percent and sterile, filtered and de-mineralized water as rest.

2. Heat transfer liquid of claim 1 for use in temperature control of the human body temperature during extracorporeal circulation, comprising ethylene glycol or propylene glycol at 30 volume-percent.

3. Heat transfer liquid of any of the preceding claims for use in temperature control of the human body temperature during extracorporeal circulation, comprising hydrogen peroxide at 0.01 volume-percent to 0.03 volume-percent, preferably 0.02 volume-percent.

4. Temperature control device for human body temperature control during extracorporeal circulation comprising heat transfer liquid according to any of claims 1 to 3.

## Patentansprüche

1. Wärmeübertragungsflüssigkeit zum Einsatz bei Temperatursteuerung der menschlichen Körpertemperatur bei extrakorporaler Zirkulation, wobei die Wärmeübertragungsflüssigkeit aus 25 Vol.-% bis 35 Vol.-% Ethylenglykol oder aus 30 Vol.-% bis 35 Vol.-% Propylenglykol, 0,01 Vol.-% bis 0,05 Vol.-% Wasserstoffperoxid sowie sterilem, gefiltertem und vollentsalztem Wasser als Rest besteht.

2. Wärmeübertragungsflüssigkeit nach Anspruch 1 zum Einsatz bei Temperatursteuerung der menschlichen Körpertemperatur bei extrakorporaler Zirkulation, die 30 Vol.-% Ethylenglykol oder Propylenglykol umfasst.

3. Wärmeübertragungsflüssigkeit nach einem der vorangehenden Ansprüche zum Einsatz bei Temperatursteuerung der menschlichen Körpertemperatur bei extrakorporaler Zirkulation, die 0,01 Vol.-% bis 0,03 Vol.-%, vorzugsweise 0,02 Vol.-% Wasserstoffperoxid umfasst.

4. Temperatursteuerungsvorrichtung für Steuerung menschlicher Körpertemperatur bei extrakorporaler Zirkulation, die Wärmeübertragungsflüssigkeit nach einem der Ansprüche 1 bis 3 umfasst.

## Revendications

1. Liquide de transfert thermique pour l'utilisation dans une régulation de température de la température du corps humain durant une circulation extracorporelle, le liquide de transfert thermique étant constitué d'éthylène glycol à 25 pour cent en volume à 35 pour cent en volume, ou de propylène glycol à 30 pour cent en volume à 35 pour cent en volume,
du peroxyde d'hydrogène à 0,01 pour cent en volume à 0,05 pour cent en volume et de l'eau stérile, filtrée et déminéralisée pour le reste.

2. Liquide de transfert thermique selon la revendication 1 pour l'utilisation dans une régulation de température de la température du corps humain durant une circulation extracorporelle, comprenant de l'éthylène glycol ou du propylène glycol à 30 pour cent en volume.

3. Liquide de transfert thermique selon l'une quelconque des revendications précédentes pour l'utilisation dans une régulation de température de la température du corps humain durant une circulation extracorporelle, comprenant du peroxyde d'hydrogène à 0,01 pour cent en volume à 0,03 pour cent en volume, préférablement à 0,02 pour cent en volume.

4. Dispositif de régulation de température pour une régulation de température du corps humain durant une circulation extracorporelle comprenant un liquide de transfert thermique selon l'une quelconque des revendications 1 à 3.
